# EUROPEAN PATENT APPLICATION

(11) **EP 1 167 072 A1**
(43) Date of publication of application: **02.01.2002**
(21) Application number: 01305644.5
(22) Date of filing: 29.06.2001
(51) Int. Cl.: B44C 1/10, B44C 1/16, B44C 1/165

(54) **Transfer**

(30) Priority: 29.06.2000 GB 0015970
(71) Applicant: Welch, Janet Mary, Rothley, Leicester LE7 7NP (GB)
(72) Inventor: Welch, Janet Mary, Rothley, Leicester LE7 7NP (GB)
(74) Representative: Stagg, Diana Christine

(57) **Abstract**

A transfer for application to skin is suitable for application to skin which has been scarred or otherwise damaged and has a design which conceals or reduces the visibility of the scar or other damage. The design is preferably a complex, multicoloured design which may be produced by a photographic or other reprographic process.

The transfer may comprise one or more component transfers and may comprise a design which closely resembles unscarred or undamaged skin.

The transfer is particularly suitable to be worn by a patient who has undergone breast surgery involving the removal of a nipple, in which case the transfer preferably includes a reproduction of a human nipple and surrounding skin.

## Description

The present invention relates to an improved transfer for application to the skin.

It is known to provide transfers for application to the skin for decorative purposes. These transfers are generally very simple in design, typically in a single colour or possibly two colours, and of a small size. They are worn in particular by young people to give the impression of a simple tattoo, but with the advantage that they are painless and quick to apply and are easily removable.

While these transfers function well for their intended purpose, they are of only limited application, because of their size and simplicity of design.

It is an object of the present invention to provide an improved transfer in which the disadvantages of the known transfers are reduced or substantially obviated.

The present invention provides a transfer for application to skin, characterised in that the transfer is suitable for application to skin which has been scarred or otherwise damaged and has a design which conceals or reduces the visibility of the scar or other damage.

In a preferred embodiment of the transfer according to the invention, the design is a complex, multicoloured design which may be produced by a photographic or other reprographic process. The transfer may comprise one or more component transfers. When the transfer comprises a plurality of separate transfers, these may be arranged by the wearer on the skin in a design which provides best cover for the scarred or otherwise damaged skin and is aesthetically pleasing.

In an alternative embodiment of the transfer according to the invention, the transfer may comprise a design which closely resembles unscarred or undamaged skin, so that when it is applied to the scarred or damaged area, the scarring or damaged is concealed or reduced in visibility and the skin appears to the observer to be relatively unscarred. In a preferred version of the transfer, where the transfer is to be worn by a patient who has undergone breast surgery involving the removal of a nipple, the transfer may include a reproduction of a human nipple and surrounding skin.

The transfer may be packaged for sale in the form of a number of component parts, together with a small test patch to check the suitability of the transfer for a particular wearer.

In a preferred embodiment of a transfer according to the invention, the transfer comprises a first skin-contacting layer, the non-skin-contacting surface of which having printed thereon the design. The printed non-skin-contacting surface of the skin-contacting layer is itself preferably coated with a protective transparent coating layer.

The skin-contacting layer is preferably in the form of a thin flexible translucent film, which adheres naturally to the skin and which shows minimal irritant/allergenic potential.

Examples of suitable materials to form the skin-contacting layer include acrylate copolymers such as butyl acrylate/octyl acryiate copolymer or methyl methacrylate/vinyl acetate /styrene copolymer; natural or synthetic latex rubber; polyvinyl butyral and styrene/ isoprene / styrene linear copolymer.

The ink used to print the design can be any suitable fully curable ink, since the ink does not contact the skin.

The design is then preferably coated with a totally transparent, flexible film, such as an acrylate or polyurethane resin film.

In a particularly preferred embodiment of a transfer according to the invention, the design is printed in the central region of the skin-contacting layer, with an unprinted border region surrounding the printed design.

The present invention further provides a method for concealing or reducing the visibility of a scar or other skin damage which method comprises the steps of applying a transfer having a design which is adapted to conceal or reduce the visibility of a scar or other damage directly onto the skin, wetting the reverse of the transfer and removing the backing layer from the transfer.

The transfer can be removed from the skin using surgical spirit.

Alternative embodiments of a transfer will now be described with reference to the accompanying drawings in which:

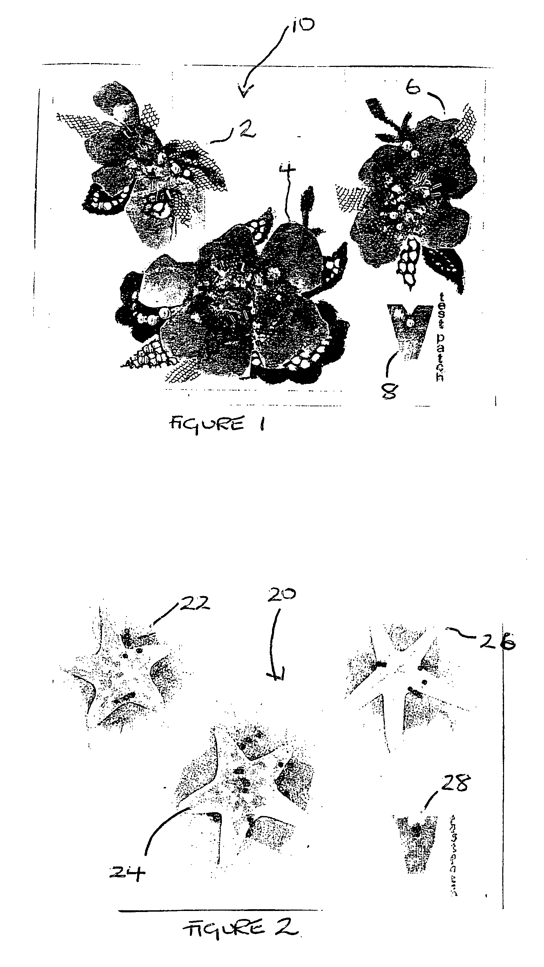

Figure 1 is a view of a first embodiment of a transfer;
Figure 2 is a view of a second embodiment of a transfer;
Figures 3a and 3b illustrate the application of a transfer and
Figure 4 is a view of a further alternative embodiment of a transfer.

As can be seen from Figurel, a sheet of transfers shown generally at 10 comprises three floral designs, 2, 4 and 6 and a test patch 8. The sheet of transfers is 160mm by 115mm in size. The three floral designs are complex in character and are extremely decorative. When applied to skin which exhibits scarring or other damage, the design acts to conceal the scarring.

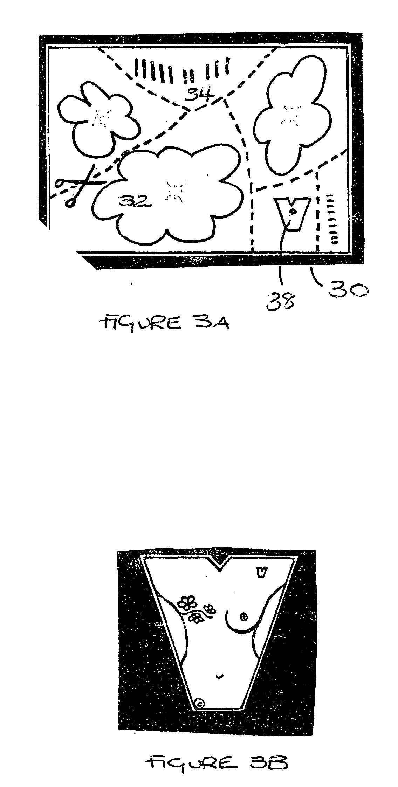

A similar product is shown in Figure 2. In this case, the transfer 20 has a design with a nautical theme, comprising shells 22, 24 and 26 and could appropriately be worn on the beach.

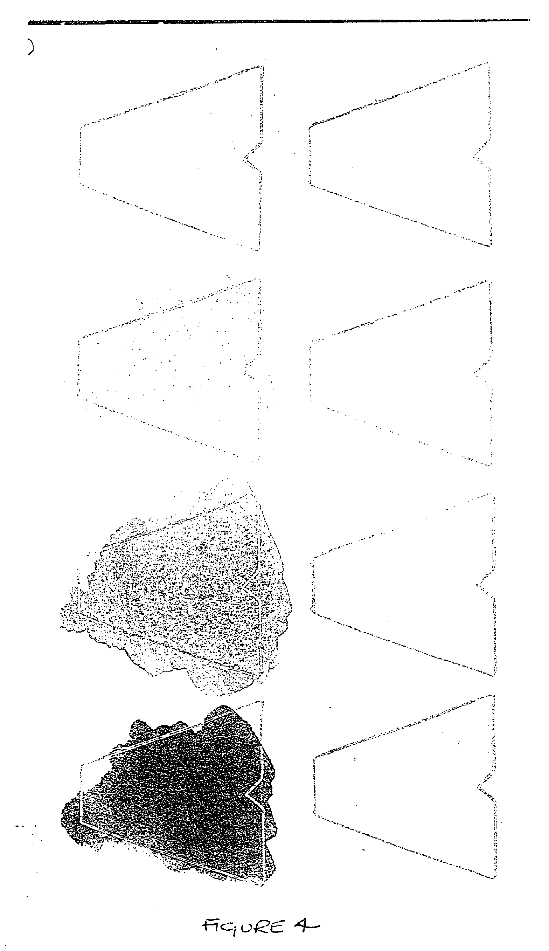

As can be seen schematically in Figure 3a, the transfer is supplied on a single sheet 30 with a number of components 32,34 and test patch 38. The transfers from sheet 30 can be applied to a person, as shown schematically in Figure 3b. In the case shown in Figure 3b, the sketch schematically illustrates a woman who has undergone a mastectomy operation. The transfers have been arranged to conceal the scarring resulting from the operation and to be aesthetically pleasing.

Figure 4 illustrates a range of transfers which closely resemble different skin tones and could be used where the wearer preferred simply to conceal the scar as completely as possible rather than to produce a decorative effect.

## Claims

1. A transfer for application to skin, **characterised in that** the transfer is suitable for application to skin which has been scarred or otherwise damaged and has a design which conceals or reduces the visibility of the scar or other damage.

2. A transfer according to claim 1 **characterised in that** the design is a complex, multicoloured design which may be produced by a photographic or other reprographic process.

3. A transfer according to claim 1 or claim 2 **characterised in that** the transfer comprises one or more component transfers.

4. A transfer according to claim 1 **characterised in that** the transfer comprises a design which closely resembles unscarred or undamaged skin.

5. A transfer according to claim 4 **characterised in that** the transfer is to be worn by a patient who has undergone breast surgery involving the removal of a nipple, and the transfer includes a reproduction of a human nipple and surrounding skin.

6. A transfer according to any of claims 1 to 5 **characterised in that** the transfer comprises a first skin-contacting layer, the non-skin-contacting surface of which having printed thereon the design.

7. A transfer according to claim 6 **characterised in that** the skin-contacting layer is in the form of a thin flexible translucent film, which adheres naturally to the skin and which shows minimal irritant / allergenic potential.

8. A transfer according to claim 7 **characterised in that** the skin-contacting layer is formed from an acrylate copolymer such as butyl acrylate / octyl acrylate copolymer or. methyl methacrylate / vinyl acetate / styrene copolymer; a natural or synthetic latex rubber; polyvinyl butyral or a styrene / isoprene / styrene linear copolymer.

9. A transfer according to any of claims 1 to 8 which is coated with a totally transparent, flexible film.

10. A transfer according to claim 9 **characterised in that** the transparent, flexible film is an acrylate or polyurethane resin film.

11. A transfer according to any of claims 6 to 10 in which the design is printed in the central region of the skin-contacting layer, with an unprinted border region surrounding the printed design.

12. A method for concealing or reducing the visibility of a scar or other skin damage which method comprises the steps of applying a transfer having a design which is adapted to conceal or reduce the visibility of a scar or other damage directly onto the skin, wetting the reverse of the transfer and removing the backing layer from the transfer.
